# EUROPEAN PATENT APPLICATION

(11) **EP 3 090 757 A1**
(43) Date of publication of application: **09.11.2016**
(21) Application number: 15166206.1
(22) Date of filing: 04.05.2015
(51) Int. Cl.: A61K 39/00, A61K 39/04

(54) **RECOMBINANT MYCOBACTERIUM AS AN IMMUNOTHERAPEUTIC AGENT FOR THE TREATMENT OF CANCER**

(71) Applicant: Vakzine Projekt Management GmbH, 30625 Hannover (DE)
(72) Inventor: Grode, Leander, 38110 Braunschweig (DE)
(74) Representative: Weiss, Wolfgang

(57) **Abstract**

The invention relates to a recombinant Mycobacterium cell for use as an immunotherapeutic agent in the treatment of cancer, particularly in the treatment of solid tumors. More particularly, the invention relates to the immunotherapy of bladder carcinoma.

## Description

The invention relates to a recombinant Mycobacterium cell for use as an immunotherapeutic agent in the treatment of cancer, particularly in the treatment of solid tumors. More particularly, the invention relates to the immunotherapy of bladder carcinoma.

Urothelial bladder carcinoma is the 5th most common cancer. In the United States, about 75.000 new cases are diagnosed each year 4.5% of all new cancers, and approximately 15.600 deaths are expected. In Germany, about 16.000 new cases are diagnosed each year. Because a recurrence of disease is likely in bladder carcinoma, patients must undergo surveillance for an extended period.

Most bladder carcinomas begin in transitional epithelial cells that make up the inner lining of the bladder. As these tumors grow, they can invade the surrounding connective tissue and muscle. In advanced disease, tumors spread beyond the bladder to nearby lymph nodes or pelvic organs or metastasize to more distant organs such as lung, liver and bone.

The overall 5-year survival rate for bladder carcinoma is 77%, and this rate has not changed significantly over the last 10 years. When considered by stage, the 5-year relative survival rates for patients with tumors restricted to the inner layer of the bladder are 96% and 69%, respectively. The rates drop to 34% for those with disease that has spread locally beyond the bladder and to 6% with distant metastases.

For patients with non-muscle invasive bladder carcinoma, treatment usually involves a surgical removal of the tumor followed by chemotherapy, usually mitomycin C, within the bladder (so-called intravesical chemotherapy). After recovering from surgery, patients with a lower risk of disease progression may undergo surveillance or additional intravesical chemotherapy. Patients with moderate-to-high-grade disease often receive intravesical immunotherapy with an attenuated live bacterium Bacillus Calmette Guérin (BCG). BCG was the first FDA-approved immunotherapy and helps reduce the risk of bladder carcinoma recurrence by stimulating an immune response that targets the bacteria as well as any bladder carcinoma cells. In some patients, however, BCG therapy has found to be less effective, particularly after repeated administration.

Standard treatment for patients with muscle-invasive bladder carcinoma includes cisplatin-based chemotherapy followed by surgical removal of the bladder or radiation therapy and concomitant chemotherapy. Recurrent bladder carcinoma may be treated with combination therapy regimens, including gemcitabine plus cisplatin or methotrexate, vinblastine, doxorubicin plus cisplatin.

In the treatment of bladder carcinoma, tumor recurrence is a major concern, even for patients with low-grade disease and requires extensive follow-up. Better treatments, such as novel immunotherapies, might reduce recurrence rates and improve the survival of patients with bladder carcinoma.

A recombinant BCG strain expressing a phagolysosomal escape domain is described in WO 99/101496, the content of which is herein incorporated by reference. The phagolysosomal escape domain enables the strain to escape from the phagosome of infected host cells by perforating the membrane of the phagosome. In order to provide an acidic phagosomal pH for optimal phagolysosomal escape activity, a urease-deficient recombinant strain was developed. This strain is disclosed in WO2004/094469, the content of which is herein incorporated.

WO 2012/085101, the content of which is herein incorporated, discloses that a recombinant Δ*ureC Hly*⁺ rBCG (rBCG) strain expressing membrane-perforating listeriolysin (Hly) of *Listeria monocytogenes* and devoid of urease C induces superior protection against aerogenic challenge with Mycobacterium tuberculosis (MTB) as compared to parental BCG (pBCG) in a preclinical model. Further, it is shown that rBCG and pBCG induce marked Th1 immune responses, whilst only rBCG elicits are profound Th17 response in addition.

In the present study, it was found that rBCG induces a superior immune response compared to pBCG in an animal model.

A subject-matter of the present invention is a recombinant Mycobacterium cell which comprises a recombinant nucleic acid molecule encoding a fusion polypeptide comprising:
(a) a domain capable of eliciting an immune response, and
(b) a phagolysosomal escape domain
for use as an immunotherapeutic agent in the treatment of solid tumors.

A further aspect of the present invention is a method for the immunotherapeutic treatment of solid tumors in a subject in need thereof, comprising administering to said subject a recombinant Mycobacterium cell which comprises a recombinant nucleic acid molecule encoding a fusion polypeptide comprising:
(a) a domain capable of eliciting an immune response, and
(b) a phagolysosomal escape domain.

According to the present invention it was found that vesicular instillation of a recombinant BCG cell into the bladder of rats surprisingly results in an increased infiltration of urinary bladder tissue by lymphocytes, particularly CD4- and CD8-positive lymphocytes resulting in a high incidence of focal and/or multifocal lymphocytic infiltration. In contrast thereto, the urinary bladder tissue of animals treated with standard BCG showed CD4- and CD8-positive lymphocytes only as single cell infiltrate levels (diffuse infiltration). Further, administration of a recombinant BCG cell did not raise any safety issues.

Thus, the present invention also relates to a recombinant Mycobacterium cell as described above for use as an immunotherapeutic agent in the treatment of solid tumors in order to obtain focal and/or multifocal lymphocytic infiltration, e.g. with CD4 and CD8 T cells at the site of administration. The recombinant Mycobacterium cell of the present invention is particularly suitable for use in human medicine.

The immunotherapeutic agent is a live recombinant Mycobacterium cell which comprises a recombinant nucleic acid molecule encoding a fusion polypeptide comprising (a) a domain capable of eliciting an immune response and (b) a phagolysosomal escape domain. The domain capable of eliciting an immune response is preferably an immunogenic peptide or polypeptide from a pathogen or an immunogenic fragment thereof.

The Mycobacterium cell is preferably an *M. bovis* cell, an *M. tuberculosis* cell, particularly an attenuated *M. tuberculosis* cell or other Mycobacteria, e.g. *M. microti*, *M. smegmatis*, *M. canettii*, *M. marinum* or M. *fortuitum*. More preferably, the cell is an attenuated recombinant *M*. *bovis* (BCG) cell, particularly a *M. bovis* BCG cell, more particularly a recombinant *M*. *bovis* BCG cell from strain Danish subtype Prague (Brosch et al., Proc. Natl. Acad. Sci. USA, 104 (2007), 5396-5601). In an especially preferred embodiment, the Mycobacterium cell is recombinant urease-deficient. In an especially preferred embodiment the ureC sequence of the Mycobacterium cell is inactivated (ΔUrec), e.g. by constructing a suicide vector containing a ureC gene disrupted by a selection marker gene, e.g. the hygromycin gene, transforming the target cell with the vector and screening for selection marker-positive cells having a urease negative phenotype. In an even more preferred embodiment, the selection marker gene, i.e. the hygromycin gene, is subsequently inactivated. In this embodiment, the cell is a selection marker-free recombinant Mycobacterium cell. Most preferably, the cell is selection marker-free recombinant BCG strain Danish subtype Prague characterized as rBCG ΔUrec :: Hly⁺.

The domain capable of eliciting an immune response is preferably selected from immunogenic peptides or polypeptides from *M. bovis, M. tuberculosis* or *M. leprae* or from immunogenic fragments thereof having a length of at least 6, preferably at least 8 amino acids, more preferably at least 9 amino acids and e.g. up to 20 amino acids. Specific examples for suitable antigens are Ag85B (p30) from *M. tuberculosis,* Ag85B (α-antigen) from *M. bovis* BCG, Ag85A from *M. tuberculosis* and ESAT-6 from *M. tuberculosis* and fragments thereof. In other embodiments, the domain capable of eliciting an immune response is selected from non-Mycobacterium polypeptides.

More preferably, the immunogenic domain is derived from the antigen Ag85B. Most preferably, the immunogenic domain comprises the sequence from aa.41 to aa.51 in SEQ ID No.2.

The recombinant nucleic acid molecule further comprises a phagolysosomal escape domain, i.e. a polypeptide domain which provides for an escape of the fusion polypeptide from the phagolysosome into the cytosol of mammalian cells. Preferably, the phagolysosomal escape domain is a Listeria phagolysosomal escape domain, which is described in US 5,733,151, herein incorporated by reference. More preferably, the phagolysosomal escape domain is derived from the listeriolysin gene (Hly) of L.monocytogenes. Most preferably, the phagolysosomal domain is encoded by a nucleic acid molecule selected from: (a) a nucleotide sequence comprising nucleotides 211 - 1722 as shown in SEQ ID No.1, (b) a nucleotide sequence which encodes for the same amino acid sequence as the sequence from (a), and (c) a nucleotide sequence hybridizing under stringent conditions with the sequence from (a) or (b).

Apart from the nucleotide sequence depicted in SEQ ID No.1 the present invention also comprises nucleic acid sequences hybridizing therewith. In the present invention the term "hybridization" is used as defined in Sambrook et al. (Molecular Cloning. A laboratory manual, Cold Spring Harbor Laboratory Press (1989), 1.101-1.104). In accordance with the present invention the term "hybridization" is used if a positive hybridization signal can still be observed after washing for one hour with 1 X SSC and 0.1 % SDS at 55°C, preferably at 62° C and more preferably at 68°C, particularly for 1 hour in 0.2 X SSC and 0.1 % SDS at 55°C, preferably at 62°C and more preferably at 68°C. A sequence hybridizing with a nucleotide sequence as per SEQ ID No.1 under such washing conditions is a phagolysosomal escape domain encoding nucleotide sequence preferred by the subject invention.

A nucleotide sequence encoding a phagolysosomal escape domain as described above may be directly obtained from a Listeria organism or from any recombinant source e.g. a recombinant E.coli cell containing the corresponding Listeria nucleic acid molecule or a variant thereof as described above.

Preferably, the recombinant nucleic acid molecule encoding for a fusion polypeptide contains a signal peptide encoding sequence. More preferably, the signal sequence is a signal sequence active in Mycobacteria, preferably in M.bovis, e.g. a native M.bovis signal sequence. A preferred example of a suitable signal sequence is the nucleotide sequence coding for the Ag85B signal peptide which is depicted in SEQ ID No.1 from nucleotide 1 to 120.

Further, it is preferred that a peptide linker be provided between the immunogenic domain and the phagolysosomal escape domain. Preferably, said peptide linker has a length of from 5 to 50 amino acids. More preferably, a sequence encoding a linker as shown in SEQ ID No.1 from nucleotide 154 to 210 or a sequence corresponding thereto as regards the degeneration of the genetic code.

The nucleic acid may be located on a recombinant vector. Preferably, the recombinant vector is a prokaryotic vector, i.e. a vector containing elements for replication or/and genomic integration in prokaryotic cells. Preferably, the recombinant vector carries the nucleic acid molecule of the present invention operatively linked with an expression control sequence. The expression control sequence is preferably an expression control sequence active in Mycobacteria, particularly in M.bovis. The vector can be an extrachromosomal vector or a vector suitable for integration into the chromosome. Examples of such vectors are known to the man skilled in the art and, for instance, given in Sambrook *et al*. supra.

The immunotherapeutic agent of the present invention is suitable for the treatment of solid tumors, such as bladder, lung, liver, breast, kidney or prostate tumors. Particularly, the present invention is suitable for the treatment of non-invasive solid tumors. In an especially preferred embodiment, the solid tumor is bladder carcinoma, e.g., non-invasive bladder carcinoma, e.g. non-invasive papillary carcinoma *in situ* (Ta), non-invasive carcinoma *in situ* (Tcis), tumor invading subepithelial connective tissue (T₁), tumor invading superficial muscle (inner half) (T2a), tumor invading deep muscle (outer half) (T2b), tumor invading perivesical tissue (T3 including T3a and T3b), tumor invading prostate, uterus or vagina (T4a), and tumor invading pelvic wall or abdominal wall (T4b). Particularly, the tumor is a superficial tumor or carcinoma *in situ* (Tcis), non-invasive papillary carcinoma (Ta), or a tumor invading subepithelial connective tissue (T1). The immunotherapeutic treatment is suitable for the treatment of primary tumors and/or for the treatment of recurring tumors.

The immunotherapeutic agent is preferably locally administered to the tumor site, i.e., to the site of a primary tumor before surgery or after surgery and optionally after chemotherapy. For the treatment of urothelial bladder carcinoma, the agent is preferably administered by vesicular instillation into the urinary bladder. For other tumors, the administration may involve local injection or, in case of lung tumors, pulmonal administration.

The immunotherapeutic agent of the invention may be administered as a first-line immunotherapy in patients, who have not been treated previously with an anti-tumor-immunotherapeutic agent such as standard BCG, or a follow-up immunotherapy in patients who have been previously treated with anti-tumor-immunotherapeutic agent such as standard BCG. The immunotherapeutic agent may be administered with a newly diagnosed solid tumor, e.g., a bladder carcinoma, or to patients, particularly patients with recurrent solid tumors, e.g., bladder carcinoma.

The immunotherapeutic agent is administered to the subject to be treated in an effective dose. For a human subject, the dose for an administration may be about 10⁷ to 10¹⁰ viable units, e.g., 10⁸ to 10⁹ viable units. Preferably, the immunotherapeutic aget is administered several times, e.g. at least 5 times up to 30 times at predetermined times during the treatment.

The immunotherapeutic agent is usually provided as a pharmaceutical preparation which comprises the recombinant Mycobacterial cell in solid form, e.g., a lyophilized or cryoconserved preparation, which is reconstituted with a suitable liquid carrier before use. Alternatively, the preparation may be provided in liquid form, e.g., as suspension.

In one embodiment, the immunotherapeutic agent of the invention is administered for the treatment of carcinoma *in situ.* A standard schedule may comprise weekly administration of the agent for at least 4, e.g., 4, 5, 6, 7 or 8 weeks as an induction therapy. The induction therapy should not start until 2-3 weeks after primary tumor surgery. After a treatment-free interval of, e.g., 4 weeks, administration may continue using maintenance therapy for at least 6 months or at least 1 year.

In a further embodiment, the immunotherapeutic agent is administered in an induction therapy in the prophylactic treatment of tumor recurrence. In this embodiment, therapy may start about 2-3 weeks after biopsy of the tumor site and be repeated, e.g., at weekly intervals for at least 4, e.g., 4, 5, 6, 7 or 8 weeks. In intermediate and high-risk tumors this may be followed by maintenance therapy.

Maintenance therapy may comprise long-term therapy, e.g., 6, 9 or 12 months therapy or even longer with treatments at monthly intervals. Alternatively, maintenance therapy may comprise 2, 3 or 4 administrations at weekly intervals, at month 3, 6, 12, 18, 24, 30 and 36.

The administration as immunotherapeutic agent of the recombinant Mycobacterium cell to site of a solid tumor as described above, may be combined with further anti-tumor therapy, e.g., radiation and/or chemotherapy. Further, the immunotherapy as described above, may be combined with a non-tumor site specific administration of the recombinant Mycobacterium cell in order to provide a general stimulation of the immune system. This non-site specific administration may be effected as described in WO 2012/085101, e.g. before surgery of the primary tumor. In this case, the agent is preferably administered to a human subject in a dose of about 1-10 x 10⁵, preferably about 2-8 x 10⁵ cells. The agent is preferably administered as a single dose, e.g., by injection. Subcutaneous injection is preferred. Further it is preferred to administer the agent without adjuvant.

Further, the invention is described in more detail by the following Figures and Examples.

### Example 1 Single Dose Toxicity Study of rBCG in Rats following Intravesicular Instillation

| **1.1 Conduct of study** | | |
|---|---|---|
| Test item | rBCG lyophilized | |
| | (rBCG Danish subtype Prague Δ Urec :: Hly+ w/o functional selection marker gene) | |
| | | |
| Approximate viable counts | 5.41×10⁸ CFU/vial | |
| | | |
| Reference item | BCG medac | |
| | | |
| Approximate viable counts | 2×10⁸ to 2×10⁹ CFU/vial | |
| | | |
| Test species / Strain / Stock | Rat / CD^{®} / Crl:CD(SD) | |
| | | |
| Breeder | Charles River Laboratories, Research Models, and Services, Germany GmbH | |
| | Sandhofer Weg 7 | |
| | 97633 Sulzfeld, Germany | |
| | | |
| Number and sex of animals | 23 female animals; | |
| | 3 animals for group 1; | |
| | 5 animals for groups 2 to 5. | |
| | | |
| Dose regime | Group 1: | Control (diluent) |
| | Group 2: | ∼2×10⁶ rBCG |
| | Group 3: | (lyophilized)/animal ∼2×10⁸ rBCG (lyophilized)/animal |
| | Group 4: | ∼2×10⁶ rBCG (frozen w/o cryoprotectant)/ animal |
| | Group 5: | ∼2×10⁶ CFU BCG medac/animal |
| | | |
| Route of administration | Intravesical instillation in the bladder | |
| Frequency of administration | Single dose on test day 1. | |
| Administration volume | 500 µL/animal | |
| | | |
| Duration of study | • 12 adaptation days | |
| | • 4 in-life test weeks | |
| | • 28 incubation days | |
| | | |

| **1.2 Results** | | |
|---|---|---|
| Mortality | None of the animals died prematurely. | |
| Clinical signs | No changes of behaviour, external appearance or condition of faeces were observed for any animal at any treatment. | |
| Body weight | The body weight of all animals of all dose groups was in the normal range throughout the course of the study. | |
| | | |
| Food and drinking water consumption | The food intake of all animals of all dose groups was in the normal range throughout the course of the study. | |
| | The visual appraisal of the drinking water consumption did not reveal any test or reference item-related influence. | |
| IL-2 levels | The IL-2 levels in urine and serum of all animals of all groups were below the lower limit of quantification. | |
| Macroscopic *post mortem* findings | No test or reference item-related changes were noted. | |
| Organ weights | No test or reference item-related changes were noted. | |
| CFU counts | rBCG (groups 2 to 4) | |
| | vs. control (group 1) | |
| | No test-item related CFU counts were noted | |
| | for the examined organs and the blood of the animals treated once with an intravesical instillation of **2×10⁶** or **2×10⁸ CFU rBCG (lyophilized)/animal,** or of **2×10⁶ CFU rBCG (frozen)/animal.** In particular, no CFU counts at all were noted for the urinary bladder four weeks after instillation of the test item, indicating a rapid clearance of the administered mycobacteria from the site of instillation. | |
| | No differences were noted between the animals treated with **2x10⁶** or **2x10⁸CFU rBCG (lyophilized)/animal** or with **2x10⁶ CFU rBCG (frozen)/animal** and the control animals. | |
| | BCG medac (group 5) vs. control (group 1) | |
| | No reference-item related CFU counts were noted for the examined organs and the blood of the animals treated once with an intravesical instillation of **2x10⁶ BCG medac/animal.** In particular, no CFU counts at all were noted for the urinary bladder four weeks after instillation of the reference item, indicating a rapid clearance of the administered mycobacteria from the site of instillation. | |
| | No differences were noted between the animals treated with **2x10⁶ BCG medac**/**animal** and the control animals. | |
| | rBCG (groups 2 to 4) | |
| | vs. BCG medac (group 5) | |
| | No difference in CFU counts was noted for the examined organs and the blood of the animals treated once with an intravesical instillation of **2x10⁶** or **2x10⁸ CFU rBCG (lyophilized) /animal,** or of **2x10⁶ CFU rBCG (frozen) /animal** compared to the reference group treated in the same way with **2x10⁶ CFU BCG medac**/**animal**. | |
| Immunohistochemistry | Immunohistochemistry for lymphocyte sub-typing in the urinary bladder tissue revealed the highest incidence of focal and multifocal lymphocytic infiltration with CD4 and CD8 positive cells in groups 2 and 3 treated once with **2x10⁶** or **2x10⁸ CFU rBCG (lyophilized)/animal** by intravesical instillation, whereas animals of groups 1 (control), 4 and 5 expressed CD4 and CD8 positive lymphocytes only as single cell infiltrate levels (diffuse infiltration). Nearly all animals (12 of 13) of groups 1 (control), 4 and 5 expressed CD4 and CD8 positive lymphocytes only as single cell | |
| | infiltrate levels (diffuse infiltration). In contrast nearly all animals (9 of 10) of groups 2 and 3 contained CD4 and CD8 positive lymphocytes in focal or multifocal lymphocyte infiltrates in addition to the diffuse infiltration. | |
| | Neutrophilic granulocytes could rarely be detected in any slides examined. | |

In conclusion, no signs of toxicity were noted for the animals treated with the test item, the reference item or the dilutent. There were no differences in the systemic spread of mycobacteria in blood and organs between **rBCG** and **BCG medac** for the treatment by intravesical instillation. No test-item related CFU counts were noted for the examined organs and the blood of the animals treated once with an intravesical instillation of **rBCG** compared to the control animals. In particular, no CFU counts at all were noted for the urinary bladder 4 weeks after instillation of the test item, indicating a rapid clearance of the administered mycobacteria from the site of instillation.

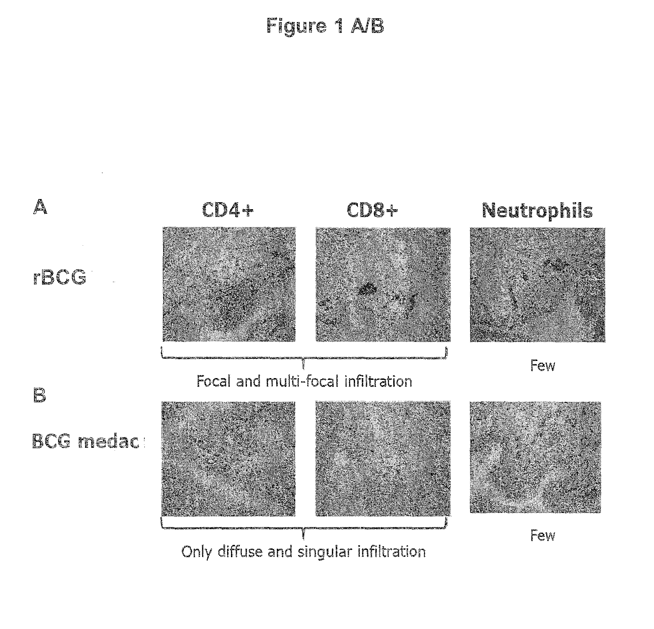

Immunohistochemistry for lymphocyte sub-typing in the urinary bladder tissue revealed the highest incidence of focal and multifocal lymphocytic infiltration with CD4 and CD8 positive cells in groups 2 and 3 treated once with **2x10⁶** or **2x10⁸ CFU rBCG/animal** by intravesical instillation, whereas animals of groups 1 (control), 4 and 5 expressed CD4 and CD8 positive lymphocytes only as single cell infiltrate levels (diffuse infiltration). Nearly all animals (12 of 13) of groups 1 (control), 4 and 5 expressed CD4 and CD8 positive lymphocytes only as single cell infiltrate levels (diffuse infiltration). In contrast, nearly all animals (9 of 10) of groups 2 and 3 contained CD4 and CD8 positive lymphocytes in focal or multifocal lymphocyte infiltrates in addition to the diffuse infiltration. Fig. 1A shows focal and multifocal lymphocytic infiltration after administration of rBCG. Fig. 1B shows only diffuse and singular infiltration after administration of BCG medac (200x magnification).

### Example 2 Repeated Dose Toxicity Study of rBCG in Rats following Intravesicular Instillation

| **2.1 Conduct of study** | | |
|---|---|---|
| Test item | rBCG lyophilized | |
| | (rBCG Danish subtype Prague Δ Urec :: Hly+ w/o functional selection marker gene) | |
| Approximate viable counts | 5.41 × 10⁸ CFU/vial | |
| Reference item | BCG medac | |
| Approximate viable counts | 2 × 10⁸ to 2 × 10⁹ CFU/vial | |
| Test species / Strain / Stock | Rat / CD^{®} / Crl:CD(SD) | |
| Breeder | Charles River Laboratories, Research Models, and Services, Germany GmbH | |
| | Sandhofer Weg 7 | |
| | 97633 Sulzfeld, Germany | |
| | | |
| Number and sex of animals | 23 female animals; | |
| | 3 animals for group 1; | |
| | 5 animals for groups 2 to 5. | |
| | | |
| Dose regime | Group 1: | Control (diluent) |
| | Group 2: | ∼2×10⁶ CFU rBCG (lyophilized)/animal |
| | Group 3: | ∼2×10⁸ CFU rBCG (lyophilized)/animal |
| | Group 4: | ∼2×10⁶ CFU rBCG (frozen w/o cryoprotectant)/animal |
| | Group 5: | ∼2×10⁶ CFU BCG medac/animal |
| Route of administration | Intravesical instillation in the bladder | |
| Frequency of administration | Repeated administration; once weekly on test days 1, 8, 15, 22, 29, and 36. | |
| Administration volume | 500 µL/animal | |
| Duration of study | • 21 adaptation days | |
| | • 9 in-life test weeks | |
| | • 28 incubation days | |
| | | |

| **2.2 Results** | | |
|---|---|---|
| Mortality | None of the animals died prematurely. | |
| Clinical signs | No changes of behaviour, external appearance or condition of faeces were observed for any animal at any treatment. | |
| Body weight | The body weight of all animals of all dose groups was in the normal range throughout the course of the study. | |
| Food and drinking water consumption | The food intake of all animals of all dose groups was in the normal range throughout the course of the study. | |
| | The visual appraisal of the drinking water consumption did not reveal any test or reference item-related influence. | |
| IL-2 levels | The IL-2 levels in urine and serum of all animals of all groups were below the lower limit of quantification. | |
| Delayed type hyper-sensitivity (DTH assay) | No delayed type hypersensitivity was noted. | |
| Macroscopic *post mortem* findings | No test or reference item-related changes were noted. | |
| Organ weights | No test or reference item-related changes were noted. | |
| CFU counts | rBCG (groups 2 to 4) vs. control (group 1) | |
| | No test-item related CFU counts were noted for the examined organs and the blood of the animals treated six times with an intravesical instillation of **2×10⁶** or **2×10⁸ CFU rBCG (lyophilized)/animal,** or of **2×10⁶ CFU rBCG (frozen/w/o cryoprotectant)/animal.** In particular, no CFU counts at all were noted for the urinary bladder four weeks after the last instillation of the test item, indicating a rapid clearance of the administered mycobacteria from the site of instillation. | |
| | No differences were noted between the animals treated with **2x10⁶** or **2x10⁸ CFU rBCG (lyophilized)/animal** or with **2x10⁶ CFU rBCG (frozen)/animal** and the control animals. | |
| | BCG medac (group 5) vs. control (group 1) No reference-item related CFU counts were noted for the examined organs and the blood of the animals treated six times with an intravesical instillation of **2×10⁶ BCG medac/animal.** In particular, no CFU counts at all were noted for the urinary bladder four | |
| | weeks after the last instillation of the reference item, indicating a rapid clearance of the administered mycobacteria from the site of instillation. | |
| | No differences were noted between the animals treated with **2x10⁶ BCG medac/animal** and the control animals. | |
| | rBCG (groups 2 to 4) | |
| | vs. BCG medac (group 5) | |
| | No difference in CFU counts was noted for the examined organs and the blood of the animals treated six times with an intravesical instillation of **2×10⁶** or **2×10⁸ CFU rBCG (lyophilized)/ animal,** or of **2×10⁶ CFU rBCG (frozen)/ animal** compared to the reference group treated in the same way with **2×10⁶ CFU BCG medac/animal.** | |
| Immunohistochemistry | Immunohistochemistry for lymphocyte sub-typing in the urinary bladder tissue revealed the highest incidence of multifocal lymphocytic infiltration with CD4 and CD8 positive cells in group 2 treated with **2×10⁶ CFU rBCG/animal** by 6 intravesical instillations, followed by group 3 treated with **2×10⁸ CFU rBCG/animal** by 6 intravesical instillations, whereas animals of groups 1 (control), 4 and 5 expressed CD4 and CD8 positive lymphocytes only as single cell infiltrate levels (diffuse infiltration). Neutrophilic granulocytes could rarely be detected in any slides examined. | |

In conclusion, no signs of toxicity were noted for the animals treated with the test item, the reference item or the diluent. There were no differences in the systemic spread of mycobacteria in blood and organs between **rBCG** and **BCG medac** for the treatment by intravesical instillation. No test-item related CFU counts were noted for the examined organs and the blood of the animals treated six times with an intravesical instillation of **rBCG** compared to the control animals. In particular, no CFU counts at all were noted for the urinary bladder 4 weeks after the last instillation of the test item, indicating a rapid clearance of the administered mycobacteria from the site of instillation.

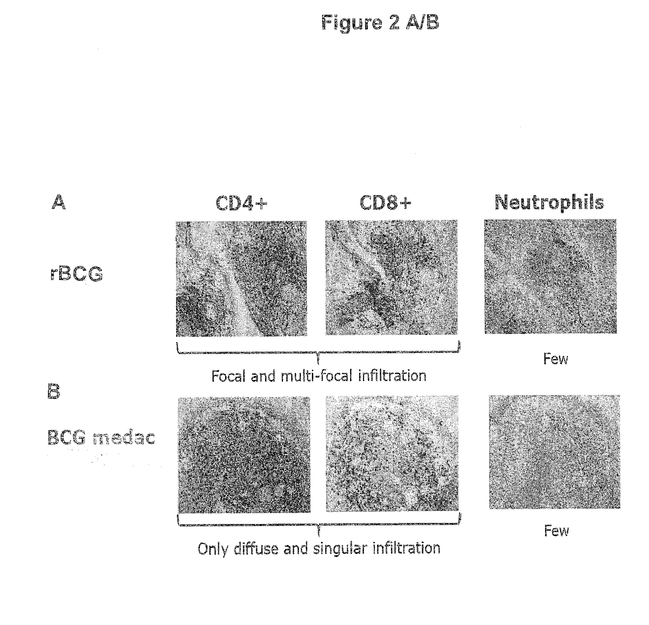

Immunohistochemistry for lymphocyte sub-typing in the urinary bladder tissue revealed the highest incidence of multifocal lymphocytic infiltration with CD4 and CD8 positive cells in group 2 treated with **2×10⁶ CFU rBCG/animal** by 6 intravesical instillations, followed by group 3 treated with **2×10⁸ CFU rBCG/animal** by 6 intravesical instillations, whereas animals of groups 1 (control), 4 and 5 expressed CD4 and CD8 positive lymphocytes only as single cell infiltrate levels (diffuse infiltration). Fig. 2A shows focal and multifocal lymphocytic infiltration after administration of rBCG. Fig. 2B shows only diffuse and singular infiltration after administration of BCG medac (200x magnification).

## Claims

1. A recombinant Mycobacterium cell which comprises a recombinant nucleic acid molecule encoding a fusion polypeptide comprising:
(a) a domain capable of eliciting an immune response, and
(b) a phagolysosomal escape domain
for use as immunotherapeutic agent in the treatment of solid tumors.

2. The cell of claim 1 which is a urease-deficient cell

3. The cell of claim 1 or 2 which is a recombinant *M*. *bovis* BCG cell, particularly a recombinant *M. bovis* BCG cell from strain Danish subtype Prague.

4. The cell of any one of claims 1-3, wherein the domain capable of eliciting an immune response is selected from immunogenic peptides or polypeptides from *M. bovis* or *M. tuberculosis.*

5. The cell of any one of claims 1-4, wherein the recombinant nucleic acid molecule does not comprise any functional selection marker.

6. The cell of any one of claims 1-5, wherein the solid tumor is bladder carcinoma.

7. The cell of any one of claims 1-5, wherein the bladder carcinoma is non-invasive bladder carcinoma, particularly carcinoma *in situ* (Tcis), non-invasive papillary carcinoma (Ta), or a tumor invading subepithelial connective tissue (T1).

8. The cell of any one of claims 1-7, wherein the treatment comprises local administration of the immunotherapeutic agent to the tumor site, particularly after surgery.

9. The cell of any one of claims 6-8, wherein the agent is administered by vesicular instillation into the urinary bladder.

10. The cell of any one of claims 1-9 for obtaining focal and/or multifocal lymphocytic infiltration at the site of administration.

11. The cell of claim 10 for obtaining focal and/or multifocal tissue infiltration with CD4 and CD8 T cells.

12. A method for the immunotherapy of solid tumors in a subject in need thereof, comprising administering to said subject a recombinant Mycobacterium cell comprising recombinant nucleic acid molecule encoding a fusion polypeptide comprising:
(a) a domain capable of eliciting an immune response, and
(b) a phagolysosomal escape domain.

13. The method of claim 12, wherein the focal and/or multifocal lymphocytic tissue infiltration is increased compared to administration of native BCG.


SEQUENCE LISTING

<110> Vakzine Projekt Management GmbH

<120> Recombinant Mycobacterium as an immunotherapeutic agent for the treatment of cancer.

<130> 60182P EP

<160> 2

<170> BiSSAP 1.0

<210> 1
<211> 1881
<212> DNA
<213> Artificial Sequence

<220>
<221> source
<222> 1..1881
<223> /mol_type="DNA"
/note="recombinant nucleic acid molecule"
/organism="Artificial Sequence"

<220>
<221> source
<222> 1..1881
<223> /mol_type="DNA"
/note="CDS comprising a phagolysosomal domain (211-1722nt) and a
stop codon (1879-1881)"
/organism="Artificial Sequence"

<400> 1
atgacagacg tgagccgaaa gattcgagct tggggacgcc gattgatgat cggcacggca 60

gcggctgtag tccttccggg cctggtgggg cttgccggcg gagcggcaac cgcgggcgcg 120

ttctcccggc cggggctgcc ggtcgagtac ctgcagtctg caaagcaatc cgctgcaaat 180

aaattgcact cagcaggaca aagcacgaaa gatgcatctg cattcaataa agaaaattca 240

atttcatcca tggcaccacc agcatctccg cctgcaagtc ctaagacgcc aatcgaaaag 300

aaacacgcgg atgaaatcga taagtatata caaggattgg attacaataa aaacaatgta 360

ttagtatacc acggagatgc agtgacaaat gtgccgccaa gaaaaggtta caaagatgga 420

aatgaatata ttgttgtgga gaaaaagaag aaatccatca atcaaaataa tgcagacatt 480

caagttgtga atgcaatttc gagcctaacc tatccaggtg ctctcgtaaa agcgaattcg 540

gaattagtag aaaatcaacc agatgttctc cctgtaaaac gtgattcatt aacactcagc 600

attgatttgc caggtatgac taatcaagac aataaaatcg ttgtaaaaaa tgccactaaa 660

tcaaacgtta acaacgcagt aaatacatta gtggaaagat ggaatgaaaa atatgctcaa 720

gcttatccaa atgtaagtgc aaaaattgat tatgatgacg aaatggctta cagtgaatca 780

caattaattg cgaaatttgg tacagcattt aaagctgtaa ataatagctt gaatgtaaac 840

ttcggcgcaa tcagtgaagg gaaaatgcaa gaagaagtca ttagttttaa acaaatttac 900

tataacgtga atgttaatga acctacaaga ccttccagat ttttcggcaa agctgttact 960

aaagagcagt tgcaagcgct tggagtgaat gcagaaaatc ctcctgcata tatctcaagt 1020

gtggcgtatg gccgtcaagt ttatttgaaa ttatcaacta attcccatag tactaaagta 1080

aaagctgctt ttgatgctgc cgtaagcgga aaatctgtct caggtgatgt agaactaaca 1140

aatatcatca aaaattcttc cttcaaagcc gtaatttacg gaggttccgc aaaagatgaa 1200

gttcaaatca tcgacggcaa cctcggagac ttacgcgata ttttgaaaaa aggcgctact 1260

tttaatcgag aaacaccagg agttcccatt gcttatacaa caaacttcct aaaagacaat 1320

gaattagctg ttattaaaaa caactcagaa tatattgaaa caacttcaaa agcttataca 1380

gatggaaaaa ttaacatcga tcactctgga ggatacgttg ctcaattcaa catttcttgg 1440

gatgaagtaa attatgatcc tgaaggtaac gaaattgttc aacataaaaa ctggagcgaa 1500

aacaataaaa gcaagctagc tcatttcaca tcgtccatct atttgccagg taacgcgaga 1560

aatattaatg tttacgctaa agaatgcact ggtttagctt gggaatggtg gagaacggta 1620

attgatgacc ggaacttacc acttgtgaaa aatagaaata tctccatctg gggcaccacg 1680

ctttatccga aatatagtaa taaagtagat aatccaatcg aatatgcatt agcctatgga 1740

agtcagggtg atcttaatcc attaattaat gaaatcagca aaatcatttc agctgcagtt 1800

ctttcctctt taacatcgaa gctacctgca gagttcgtta ggcgcggatc cggaattcga 1860

agcttatcga tgtcgacgta g 1881

<210> 2
<211> 626
<212> PRT
<213> Artificial Sequence

<220>
<221> SOURCE
<222> 1..626
<223> /mol_type="protein"
/note="CORRESPONDING AMINO ACID SEQUENCE OF SEQ ID NO: 1."
/organism="Artificial Sequence"

<400> 2
Met Thr Asp Val Ser Arg Lys Ile Arg Ala Trp Gly Arg Arg Leu Met
1               5                   10                  15
Ile Gly Thr Ala Ala Ala Val Val Leu Pro Gly Leu Val Gly Leu Ala
            20                  25                  30
Gly Gly Ala Ala Thr Ala Gly Ala Phe Ser Arg Pro Gly Leu Pro Val
        35                  40                  45
Glu Tyr Leu Gln Ser Ala Lys Gln Ser Ala Ala Asn Lys Leu His Ser
    50                  55                  60
Ala Gly Gln Ser Thr Lys Asp Ala Ser Ala Phe Asn Lys Glu Asn Ser
65                  70                  75                  80
Ile Ser Ser Met Ala Pro Pro Ala Ser Pro Pro Ala Ser Pro Lys Thr
                85                  90                  95
Pro Ile Glu Lys Lys His Ala Asp Glu Ile Asp Lys Tyr Ile Gln Gly
            100                 105                 110

Leu Asp Tyr Asn Lys Asn Asn Val Leu Val Tyr His Gly Asp Ala Val
        115                 120                 125
Thr Asn Val Pro Pro Arg Lys Gly Tyr Lys Asp Gly Asn Glu Tyr Ile
    130                 135                 140
Val Val Glu Lys Lys Lys Lys Ser Ile Asn Gln Asn Asn Ala Asp Ile
145                 150                 155                 160
Gln Val Val Asn Ala Ile Ser Ser Leu Thr Tyr Pro Gly Ala Leu Val
                165                 170                 175
Lys Ala Asn Ser Glu Leu Val Glu Asn Gln Pro Asp Val Leu Pro Val
            180                 185                 190
Lys Arg Asp Ser Leu Thr Leu Ser Ile Asp Leu Pro Gly Met Thr Asn
        195                 200                 205
Gln Asp Asn Lys Ile Val Val Lys Asn Ala Thr Lys Ser Asn Val Asn
    210                 215                 220
Asn Ala Val Asn Thr Leu Val Glu Arg Trp Asn Glu Lys Tyr Ala Gln
225                 230                 235                 240
Ala Tyr Pro Asn Val Ser Ala Lys Ile Asp Tyr Asp Asp Glu Met Ala
                245                 250                 255
Tyr Ser Glu Ser Gln Leu Ile Ala Lys Phe Gly Thr Ala Phe Lys Ala
            260                 265                 270
Val Asn Asn Ser Leu Asn Val Asn Phe Gly Ala Ile Ser Glu Gly Lys
        275                 280                 285
Met Gln Glu Glu Val Ile Ser Phe Lys Gln Ile Tyr Tyr Asn Val Asn
    290                 295                 300
Val Asn Glu Pro Thr Arg Pro Ser Arg Phe Phe Gly Lys Ala Val Thr
305                 310                 315                 320
Lys Glu Gln Leu Gln Ala Leu Gly Val Asn Ala Glu Asn Pro Pro Ala
                325                 330                 335
Tyr Ile Ser Ser Val Ala Tyr Gly Arg Gln Val Tyr Leu Lys Leu Ser
            340                 345                 350
Thr Asn Ser His Ser Thr Lys Val Lys Ala Ala Phe Asp Ala Ala Val
        355                 360                 365
Ser Gly Lys Ser Val Ser Gly Asp Val Glu Leu Thr Asn Ile Ile Lys
    370                 375                 380
Asn Ser Ser Phe Lys Ala Val Ile Tyr Gly Gly Ser Ala Lys Asp Glu
385                 390                 395                 400
Val Gln Ile Ile Asp Gly Asn Leu Gly Asp Leu Arg Asp Ile Leu Lys
                405                 410                 415
Lys Gly Ala Thr Phe Asn Arg Glu Thr Pro Gly Val Pro Ile Ala Tyr
            420                 425                 430
Thr Thr Asn Phe Leu Lys Asp Asn Glu Leu Ala Val Ile Lys Asn Asn
        435                 440                 445
Ser Glu Tyr Ile Glu Thr Thr Ser Lys Ala Tyr Thr Asp Gly Lys Ile
    450                 455                 460
Asn Ile Asp His Ser Gly Gly Tyr Val Ala Gln Phe Asn Ile Ser Trp
465                 470                 475                 480
Asp Glu Val Asn Tyr Asp Pro Glu Gly Asn Glu Ile Val Gln His Lys
                485                 490                 495
Asn Trp Ser Glu Asn Asn Lys Ser Lys Leu Ala His Phe Thr Ser Ser
            500                 505                 510
Ile Tyr Leu Pro Gly Asn Ala Arg Asn Ile Asn Val Tyr Ala Lys Glu
        515                 520                 525
Cys Thr Gly Leu Ala Trp Glu Trp Trp Arg Thr Val Ile Asp Asp Arg
    530                 535                 540
Asn Leu Pro Leu Val Lys Asn Arg Asn Ile Ser Ile Trp Gly Thr Thr
545                 550                 555                 560
Leu Tyr Pro Lys Tyr Ser Asn Lys Val Asp Asn Pro Ile Glu Tyr Ala
                565                 570                 575
Leu Ala Tyr Gly Ser Gln Gly Asp Leu Asn Pro Leu Ile Asn Glu Ile
            580                 585                 590
Ser Lys Ile Ile Ser Ala Ala Val Leu Ser Ser Leu Thr Ser Lys Leu
        595                 600                 605
Pro Ala Glu Phe Val Arg Arg Gly Ser Gly Ile Arg Ser Leu Ser Met

    610                 615                 620
Ser Thr
625